Europäisches Patentamt

European Patent Office

Office européen des brevets

(11) Publication number: **0 441 123 A1**

# EUROPEAN PATENT APPLICATION

(21) Application number: 91100227.7

(22) Date of filing: 09.01.91

(51) Int. Cl.⁵: **A61L 27/00**, A61L 31/00,
A61L 15/26, A61L 15/24,
A61L 15/64

(30) Priority: 06.02.90 US 475564

(43) Date of publication of application:
**14.08.91 Bulletin 91/33**

(84) Designated Contracting States:
**BE CH DE DK ES FR GB GR IT LI NL SE**

(71) Applicant: **AMERICAN CYANAMID COMPANY**
**1937 West Main Street P.O. Box 60**
**Stamford Connecticut 06904-0060(US)**

(72) Inventor: **Landi, Henry P.**
**603 Glenwood Road**
**Yorktown Heights, New York 10598(US)**

(74) Representative: **Wächtershäuser, Günter, Dr.**
**Tal 29**
**W-8000 München 2(DE)**

(54) **Composite material having absorbable and nonabsorbable components.**

(57) The invention is a composite material of two or more biocompatible polymers, at least one of which is polytetrafluoroethylene (PTFE) and one of which is a bioabsorbable polymer. The nonabsorbable PTFE is used in the composite as a reinforcing binder. The reinforcing binder is a network of unsintered, interconnected microfibers which are formed, for example, by blending with a thermoplastic polymer vehicle, such as polymethylmethacrylate which is subsequently extracted. The bioabsorbable component is contained within the structure of the PTFE microfibrils. This composite is useful in the repair of mammalian tissue where tissue ingrowth and permanent support is required.

## COMPOSITE MATERIAL HAVING ABSORBABLE AND NONABSORBABLE COMPONENTS

This invention relates to a composite of two or more biocompatible polymers, at least one of which is polytetrafluoroethylene (PTFE) and the other component being a bioabsorbable polymer. The nonabsorbable PTFE is used in the composite as a reinforcing binder. The reinforcing binder is a network of unsintered, interconnected microfibers. The microfibers can be formed, for example, by blending with a thermoplastic polymer vehicle, such as polymethylmethacrylate. The bioabsorbable polymer component can be present in the form of a particulate fiber contained within the PTFE microfibrillar structure. The bioabsorbable particulate component can be micropulverized and could be, for example, polyglycolic acid (PGA), polylactic acid (PLA), a homo- or copolymer of trimethylene carbonate (TMC), and blends of the same or similar polymers. The polymethylmethacrylate is subsequently extracted with a suitable solvent. The resulting microporous structure has a tortuous porosity.

Alternatively, the bioabsorbable polymer can serve as the thermoplastic vehicle during the PTFE fibrillation process thus resulting in a nonporous reinforced composite structure. The bioabsorbable polymer vehicle component could be a copolymer of polyglycolic acid and trimethylene carbonate (GLY/TMC) or other bioabsorbable thermoplastic.

The composite described in this application may be useful in many biomedical applications, such as a tissue, hernia, or ligament repair device, a burn or wound dressing, a pledget, a drug delivery system and a tubular article, for example a vascular graft. The bioabsorbable component enhances tissue ingrowth within the fibrillar PTFE matrix. The nonabsorbable microfibrillar PTFE matrix provides additional support, direction and strength to the natural tissue formation.

The composite polymer structure described in this invention is useful in biomedical applications, such as in tissue repair, hernia repair, ligament repair, burn and wound dressing, pledgets, drug delivery systems, vascular grafts, etc. The bioabsorbable component enhances tissue ingrowth within the fibrillar PTFE matrix, which provides additional support, direction and strength to the natural tissue formation.

An advantage of this invention is that since a thermoplastic vehicle is used to form the fibrillar PTFE matrix, it therefore can be extruded into many different shapes such as a rod, tube, tape, film, or other intricate forms before extracting the thermoplastic.

Another advantage is that the PTFE is unsintered. Thus, the break down temperature of known bioabsorbable polymers (e.g., less than about 250° C) is avoided in the processing of the composite of this invention.

The completely fibrillated, unsintered polytetrofluoroethylene (PTFE) reinforcing binder of this invention has advantages over a prior art porous, sintered PTFE product. Some of the advantages of this invention, in summary form, are as follows. A filler can be added directly to the PTFE polymer without subjecting it to the detrimental effects of the sintering temperature (approximately 325° F) of PTFE. Also, a level as high as 97% filler content in the unsintered PTFE polymer can be achieved.

The prior art describes methods of preparation and nonbiological uses of fibrillar PTFE. The nonbiological uses include electrochemical applications.

A composite material for use with mammalian tissue has been invented. The composite material comprises:

a) an unsintered, microfibrillar, nonabsorbable biocompatible component prepared from polytetrafluoroethylene, and

b) a bioabsorbable component manufactured from a polymer prepared from one or more monomers selected from the group consisting of lactides, carbonates, oxalates and lactones, and optionally

c) a nonabsorbable, biocompatible thermoplastic component manufactured from a polymer which is liquid at a temperature from about 150 to 200° C and solid at ambient temperature, and which provides additional integrity to the unsintered component. In one embodiment, the bioabsorbable component is selected from the group consisting of lactides, carbonates and lactones. In a specific embodiment, the lactides are selected from the group consisting of glycolide and 3,6-dimethyl-1,4-dioxane-2,5-dione; the carbonate is 1,3-dioxan-2-one; and the lactones are selected from the group consisting of e-caprolactone and 1,4-dioxan-2-one. In another embodiment, the bioabsorbable component is manufactured from glycolide.

In combination with any of the above embodiments, other embodiments are the bioabsorbable component enmeshed in the pores of the unsintered component; the composite material in the form of a sheet or a hollow tube; and the nonabsorbable, thermoplastic component being poly(ethylenevinylacetate).

Still another embodiment is wherein the nonabsorbable, thermoplastic component is enmeshed in the pores of the unsintered component. In a specific embodiment, the nonabsorbable, thermoplastic component is poly(ethylenevinyl acetate).

An alternative composite material for use with mammalian tissue has also been invented. The alternative composite material comprises:

a) an unsintered, microfibrillar, non-absorbable biocompatible component prepared from polytetrafluoroethylene, and

b) a particulate bioabsorbable component manufactured from a polymer prepared from one or more monomers selected from the group consisting of lactides, carbonates, oxalates and lactones, and optionally

c) a nonabsorbable, biocompatible thermoplastic component manufactured from a polymer which is liquid at a temperature from about 150 to 200°C and solid at ambient temperature, and which provides additional integrity to the unsintered component. In one embodiment, the particulate bioabsorbable component is selected from the group consisting of lactides, carbonates and lactones. In a specific embodiment, the lactides are selected from the group of glycolide and 3,6-dimethyl-1,4-dioxan-2,5-dione; the carbonate is 1,3-dioxan-2-one; and the lactones are selected from the group consisting of e-caprolactone and 1,4-dioxan-2-one. In another embodiment, the bioabsorbable component is manufactured from glycolide.

In combination with any of the above embodiments, other embodiments are the particulate bioabsorbable component being micropulverized; the composite material in the form of a sheet or a hollow tube; and the nonabsorbable, thermoplastic component being poly(ethylenevinyl acetate).

Another alternative composite material for use with mammalian tissue has also been invented. The other alternative composite material comprises a first part consisting of the two component and optional three component composite material described above; and a second part affixed to at least one side of the first part. The second part comprises a bioabsorbable textile reinforcement component. The bioabsorbable reinforcement component is woven or knitted, and is manufactured from the same or a different polymer than the bioabsorbable component of the first part. In one embodiment, the bioabsorbable textile reinforcement component is selected from the group consisting of lactides, carbonates and lactones. In a specific embodiment, the lactides are selected from the group consisting of glycolide and 3,6-dimethyl-1,4-dioxan-2,5-dione; the carbonate is 1,3-dioxan-2-one; and the lactones are selected from the group consisting of e-caprolactone and 1,4-dioxan-2-one. In another embodiment, the textile reinforcement material is affixed to both sides of the first part. In a specific embodiment, the reinforcement material is laminated to the first part.

A drawing which describes the shape and/or geometrical configuration of the composite material is not necessary for an understanding of this invention. That is, any person skilled in the composite art will know how to manufacture and how to use the invention by reading this specification generally, and the examples specifically.

## DESCRIPTION

The material contains a fibrillated form of polytetrafluoroethylene (PTFE). It is processed without fusing the polymer at its sintering temperature (about 327°C). It is a porous, membrane-like structure which is formed by mixing micro-particles of PTFE with a molten thermoplastic polymer, such as polymethylmethacrylate. The thermoplastic blend is then formed into a film, or other configuration, and the thermoplastic phase is subsequently extracted with a suitable solvent. The resultant pliable unsintered, fibrillar PTFE has a tortuous porous structure and is an excellent binder for many functional fillers.

One or more bioabsorbable polymers PGA fibers and/or micropulverized PGA powder can be incorporated into this porous PTFE fibrillar matrix. This bioabsorbable filler could enhance tissue ingrowth within its inherent tortuous porous structure. Therefore, it would be useful in ligament replacement and repair, membrane organ separators, burn or wound dressings, hernia repair, pericardial substitute, etc.

Some changes could be made in the preparation of the film to reduce the fabricating temperatures below 100°C. This might include the use of extractable polymers or other extractable vehicles other than polymethylmethacrylate. Also, the coagulation of the PTFE dispersion as described in the prior art results in a viscous mass which might be milled and fabricated to form a completely fibrous PTFE sheet similar to those prepared by the present process. Other improvements in the process might include the use of water soluble polymers such as polyethylene oxide.

Various fillers can be incorporated into this film to levels as high as 97%. As a filled, coated, or impregnated material, this unique porous, fibrous, unsintered PTFE offers a vast number of possibilities related to the combined properties of PTFE in this form with those of its filler, coating, etc. The use of additional extractable fillers, for example colloidal silica, to modify the structure is disclosed in the prior art.

The fibrillar PTFE composite may be modified by combination with other reinforcing agents, such as meshes, woven fabrics, knitted fabrics, etc. prepared from biocompatible polymers. Also, the PTFE (in the micropulverized or dispersion form before fibrillating) can be incorporated directly into a bioabsorbable thermoplastic polymer, such as a homopolymer of polyglycolic acid (PGA), or a copolymer of PGA, e.g. one containing glycolic acid ester and trimethylene carbonate linkages (GLY/TMC), thus forming the microfibrillar structure in situ and eliminating the extractable thermoplastic vehicle. The fibrillar PTFE reinforced bioabsorbable polymer may also be extruded as a fiber for use as a suture, woven fabric, etc.

The thermoplastic vehicle may be removed such as by extraction with a suitable solvent. The remaining unsintered, fibrillar PTFE has a tortuous, microporous structure which is an excellent binder at levels as low as 1% for many functional fillers including bioabsorbable materials such as PGA, GLY/TMC and polydioxanone. The novel feature of this invention is the addition of a bioabsorbable filler which enhances tissue ingrowth within the tortuous, microporous fibrillar structure of the non-absorbable PTFE, which acts as reinforcement for the new tissue growth.

This fibrous, unsintered PTFE may be used as a carrier, reinforcing agent, or binder for other polymeric materials, both thermoplastic or thermoset, incorporated directly or by post-impregnation to further enhance the composite structure and degree of porosity (from non-porous to highly porous).

A matrix and/or its performance in fuel cells can be conveniently prepared in the following prior art examples 1 to 7, which are not to be taken as limitative of the invention. Unless otherwise stated, the parts given are by weight.

## Example 1

A blend of (a) 20% by weight, of polytetrafluoroethylene in the form of an aqueous dispersion containing 59%-61% polytetrafluoroethylene solids and 5.5%-6.5%, by weight based on weight of said solids, of an octylphenolpolyoxyethylene and (b) 80% of polymethylmethacrylate, by weight based on the overall solids, is milled on preheated rolls at 170° C.-175° C. During the milling operation, the polytetrafluoroethylene particles form lengthy interwoven, interconnected fibrils or fibers. A 1/8 inch x 2 inch x 4 inch plaque is next formed by injection molding the above blend and compressing the same between caul plates for from 5 to 10 minutes at 160° C.-170° C. and 3000 p.s.i.g. This plaque is cooled to room temperature and released from the mold.

The formed sheet measures approximately eight inches in diameter and from ten to twenty mils in thickness. The sheet is soaked several times in acetone to dissolve the polymethylmethacrylate present in the sheet. It is then rinsed with alcohol, washed several times with deionized water and, finally, dried by rolling between blotter paper.

## Example 2

Polymethylmethacrylate (95 parts) is heated and milled to a molten viscous state on a rubber mill maintained at a temperature between 170° C. and 190° C. To the rubber mill are next added 5 parts of polytetrafluoroethylene in the form of a 60% aqueous emulsion and 95 parts of ceric oxide are blended into the molten polymethylmethacrylate. On cooling the blend, resultant solid is ground into pellets and injection molded into a 1/8 inch x 2 inch x 4 inch plaque. The plaque is next compression molded at temperatures between 180° C. and 200° C. at a pressure of about 1000 p.s.i. into a sheet 30 mils thick and 6 inches square. This sheet is then immersed in acetone for sixteen hours at 25° C. so as to extract polymethylmethacrylate therefrom. Thereafter, the sheet is washed with acetone for from one to two hours in subsequent washings.

Resultant sheet is then washed with ethyl alcohol and water. After all the polymethylmethacrylate is removed, the sheet is composed substantially of 95% of ceric oxide and 5% of completely fibrillated polytetrafluoroethylene. The sheet is then completely saturated with water which displaces the ethyl alcohol. The sheet is next immersed in a 30% aqueous potassium hydroxide electrolyte solution until completely saturated.

Good performance of an hydrogen-oxygen fuel cell is noted when the above prepared matrix is utilized at temperatures even as high as 200° C.

## Example 3

Following the procedure of Example 2 above in every detail, except that the sheet will contain 95%, by weight, of tin acid phosphate in lieu of 95% ceric oxide. The so formed sheet is saturated with water in the same manner and, subsequently, is vacuum impregnated with 100% phosphoric acid until saturated.

Resultant impregnated sheet is then cut three inches square and placed between 2 inch square electrodes. The compressed laminate is inserted in a hydrogen/oxygen fuel cell which is operated at 175° C. with good attendant performance. Current density and operating voltage data are recorded in Table I below.

<u>Table I</u>

| Operating voltage (in volts): | | Current density, ma./cm.$^2$ |
|---|---|---|
| 0.970 | ........................ | Open circuit |
| 0.927 | ........................ | 10 |
| 0.863 | ........................ | 40 |
| 0.756 | ........................ | 100 |
| 0.596 | ........................ | 200 |

The internal resistance of the cell measures 0.014 ohm at 175° C.

Substituting for the tin acid phosphate in the above example a sodium-etched polytetrafluoroethylene floc, there is prepared a matrix possessing similarly enhanced performance characteristics.

Example 4

A sheet containing 80% by weight of barium sulfate and 20% by weight of unsintered, completely fibrillated polytetrafluoroethylene is prepared as described above in Example 2. The porous, non-electron conducting filled sheet is then washed successively with alcohol, and then with water and, finally, is saturated with 100% phosphoric acid. Resultant sheet is cut to size and placed between two standard platinized carbon water-proofed electrodes and tested as a hydrogen-air fuel cell at 175° C. with good performance. The data is summarized in Table II below.

<u>Table II</u>

| Operating voltage: | | Current density, ma./cm.$^2$ |
|---|---|---|
| 0.966 | ........................ | Open circuit |
| 0.885 | ........................ | 10 |
| 0.745 | ........................ | 40 |
| 0.533 | ........................ | 100 |

The internal resistance of the cell measures 0.059 ohm at 175° C. Example 5

This example illustrates the effect on porosity and tensile strength of sheets prepared by the procedure of Example 2 incorporation into unsintered, porous, extensively fibrillated polytetrafluoroethylene material a variety of nonelectron conducting fibers in varying proportions which are summarized in Table III below as Sheets A through D. There is also included as Sheet E, the unfilled, porous, unsintered, extensively fibrillated sheet prepared in accordance with Example 1, and as Sheet F, commercially available sintered polytetrafluoroethylene sheet. In Table III below, there are summarized compositions with and without filler prepared in accordance with the procedure set forth in Example 2 above.

## Table III

| Sheet | Percent Filler (by weight) | % (by weight) of the completely fibrillated, un-sintered poly-tetrafluoro-ethylene, percent |
|---|---|---|
| A | Polymonochlorotrifluoroethylene, 26 . | 74 |
| B | Polymonochlorotrifluoroethylene, 90 . | 10 |
| C | Ceric Oxide, 95...................... | 5 |
| D | Barium Sulfate, 80 ................... | 20 |
| E | 0 ................................... | 100 |
| F | 0 ................................... | 100 |

Porosity and tensile strength data for each of the typical filled and unfilled Sheets A through F above are summarized in Table IV below.

## TABLE IV

| Sheet | Porosity | | | | Tensile Properties | |
|---|---|---|---|---|---|---|
| | Total Porosity (Vol. %) | Volume percent of pores within the range of— | | | Tensile Modulus of Elasticity, p.s.l. | Percent Elongation to break |
| | | $<0.035\mu$ | $0.1–1.0\mu$ | $1.0–10.0\mu$ | | |
| A | 43.0 | 3.66 | 39.0 | 20.1 | 35,000 | 12.5 |
| B | 37.5 | 6.25 | 67.0 | 15.0 | 28,000 | 57.4 |
| C | 61.0 | 18.1 | 48.0 | 16.4 | 14,000 | 20.0 |
| D | 46.5 | 12.1 | 67.0 | 6.0 | 17,000 | 11.6 |
| E | 46.34 | ---------- | 32.3 | 34.8 | [1] 33,000 | [2] 15.9 |
| F | 47.4 | 9.5 | 12 | 65 | 4,000 | 8.1 |

[1] Yielded before breaking.
[2] At yield.

It can be readily seen from the above table that the highly porous, completely fibrillated, unsintered polytetrafluoroethylene of the invention functions as an inert binder or support for large quantities of a variety of nonelectron conducting fillers. Advantageously, enhanced structures which exhibit unexpected good tensile strength properties are thereby obtained.

Example 6

A paste mixture of 8 parts of platinum black, 2 parts of colloidal alumina and 3 parts by volume of polytetrafluoroethylene emulsion (60% solids) is mixed with 6 parts by volume of water and 4 parts by volume of mineral oil and rolled onto a 6 inch x 10 inch screen of expanded tantalum metal. The resulting structure contains 20 mg./cm.$^2$ of platinum. The rolling operation requires 10 to 15 passes at 300 pounds per linear inch until the paste becomes hard and then at 650 pounds per linear inch for the remaining passes until the hard paste is perfectly imbedded into the expanded metal screen. The structure is washed for 1.5 hours in heptane at 80° C., then for 0.5 hour in 2B alcohol at room temperature, rinsed with water to remove the alcohol, followed by soaking in $6NH_2SO_4$ for 1.5 hours at 80° C. to remove colloidal alumina and, finally rinsed with distilled water and dried on filter paper.

The catalyzed screen so prepared is laminated with a sheet of 0.010 inch thick porous, unsintered, fibrillated polytetrafluoroethylene as hereinabove prepared by pressing between caul plates at 500 p.s.i. for ten minutes at 150° C. To the electrode structure is spot welded a platinum current collector onto the edge of the expanded tantalum screen.

The laminated electrode structure is then incorporated into a free electrolyte cell. The electrodes have an exposed area of five square centimeters and the interelectrode spacing is 1/8 inch as defined by the thickness of the electrolyte block. The latter is filled with 85% phosphoric acid, and the cell assembly is heated to 150° C. At this temperature, the cell resistance is 0.16 ohm. Over a prolonged time period, no leakage of electrolyte occurs, although the porous, unsintered fibrillated polytetrafluoroethylene (TFE) backing is 0.010 inch thin.

Electrodes prepared having the porous, fibrillated polytetrafluoroethylene backing are tested as hydrogen and propane anodes in hydrogen-oxygen and propane-oxygen fuel cells, respectively. The oxygen electrode in these tests contained the same catalyst paste composition, but commercially available porous polytetrafluoroethylene sheets (0.04 inch thick) prepared by sintering are substituted for the porous, unsintered, fibrillated polytetrafluoroethylene. Overall operating results for hydrogen-oxygen and propane-oxygen fuel cells with 85% phosphoric acid are recorded in Table V below.

### TABLE V

| Current density, ma. [1]/cm. [2] | Operating voltage (150° C.) | |
|---|---|---|
| | Hydrogen/$O_2$ | Propane/$O_2$ |
| 0 | 1.00 | 0.90 |
| 10 | 0.95 | 0.62 |
| 20 | 0.92 | 0.55 |
| 40 | 0.88 | 0.47 |
| 100 | 0.78 | 0.31 |
| 200 | 0.62 | 0.13 |

[1] Ma.=milliamperes.

In comparing the foregoing hydrogen and propane electrodes with electrodes prepared with the same catalyst paste composition of Example 6, but substituting a commercially available sintered, porous polytetrafluoroethylenen sheet of 0.04 inch thickness, the following fuel cell results are reported in Table VI below.

### TABLE VI

| Current density, ma.[1]/cm.[2].: | Operating voltage (150° C.) propane/$O_2$ |
|---|---|
| 0 | 0.91 |
| 10 | 0.61 |
| 20 | 0.53 |
| 40 | 0.44 |
| 100 | 0.27 |
| 200 | 0 |

[1] Ma.=milliamperes.

Example 7

95 parts of polymethylmethacrylate are heated and milled to a molten viscous state on a rubber mill maintained at a temperature between 170° C. and 175° C. 5 parts of polytetrafluoroethylene as a 60% aqueous emulsion and 20 parts of graphite obtained as a byproduct in the manufacture of calcium cyanamide are blended into the molten polymethylmethacrylate. Upon cooling of the blend, the latter is ground into pellets and injection molded into a 1/8 inch x 2 inch x 4 inch plaque. The plaque is next compression molded at temperatures between 180° C. and 200° C. and at a pressure of about 1000 p.s.i. into a sheet 30 mils thick and 6 inches square. The latter sheet is then immersed in acetone for 16 hours at 25° C. so as to extract polymethylmethacrylate therefrom. Thereafter, the sheet is washed with acetone for 1 to 2 hours in two subsequent washings. This sheet is dark gray in color, flexible and is found to be from 9 to 11 mils thick.

A portion of the above-prepared sheet is impregnated with a 10% solution of chloroplatinic acid in ethanol and heated to remove solvent. The dry sheet is recovered on solvent removal. It is then subjected to nitrogen at 300° C. and then to hydrogen at 100° C. for 30 minutes so as to reduce the chloroplatinic acid to platinum. A catalyzed electrode containing 1.3 milligrams of platinum per square centimeter is obtained.

One inch diameter discs are cut from the so prepared electrode sheet and tested both as an hydrogen and as an oxygen electrode against a 9 milligram platinum per square centimeter standard electrode in matrix fuel cells using 5 N potassium hydroxide and 5 N sulfuric acid electrolyte at 70° C. The results of these tests are shown in Table VII below.

TABLE VII

| Matrix Having Electrolyte As— | Current Density (ma./cm.²) | | | | | |
|---|---|---|---|---|---|---|
| | As an H₂— Electrode at— | | | As an O₂— Electrode at— | | |
| | .85 volt | .80 volt | .75 volt | .85 volt | .80 volt | .75 volt |
| 5 N H₂SO₄ | 8 | 18 | 40 | | | 2 |
| 5 N KOH | 70 | 100 | 130 | 40 | 140 | 200 |

Example 8

A microporous bicomponent composite structure of fibrillated PTFE semi-permeable membrane sheet containing high levels of micropulverized PGA polymer is prepared as follows:

On a rubber mill are heated to about 160°-170° C. a molten blend of the following ingredients, listed in order of addition, and mixed thoroughly:

| Blend No. | Components | Wt., g. |
|---|---|---|
| 1 | Acrylite™ H-12 | 200 |
| | 33.2 ml of PTFE in aqueous suspension - DuPont 30B | 30 PTFE |
| | PGA micropulverized to about 150 microns | 170 |

The micropulverized PGA powder is formed by grinding the polymer pellets in a mill mixed with dry ice to prevent overheating. The resultant ground powder is sieved through a 100 mesh screen to recover the micropulverized powder of about 150 microns or less.

The blend was removed from the rubber mill in one uniform sheet. While the blended sheet was still hot from the mill, it was cut into 8 pieces of approximately equal size to be used for compressing into thinner sheets of film.

A disc was compressed from one of the pieces of Blend #1 between stainless steel caul plates and shim stock of 24 mils (0.024 inch) thick for about 15 min. at 350°F-360°F at a pressure of 30 tons on a 6 inch diameter ram. The platens of the molding press measured 12 inches X 12 inches in size.

The molded disc was removed from between the heated platens and cooled in another press under pressure for about 5 min.

The resultant disc of blended material measured approximately 10 inches in diameter, 24 mils (0.024 inch) thick, and weighed approximately 50 g.

The disc was subsequently immersed in acetone solvent to extract the PMMA (Acrylite™ polymer, American Cyanamid Company, NJ, USA) phase. The resultant composite film structure is microporous and contains the remaining unextractable polymers, that is, PGA (85 wt. %) and fibrillated PTFE (15 wt. %) binder.

Example 9

The following tricomponent blend is prepared similarly to Example 8.

A microporous fibrillated PTFE containing PEVA and high levels of micropulverized PGA polymer is blended on a rubber mill preheated to about 160-170° C. The composition of the blend is as follows:

| Blend No. | Components | Wt. g. |
|---|---|---|
| 2 | Acrylite™ H-12 | 200 |
| | PTFE (DuPont 30B susp.) | 30 |
| | Poly(ethylene-Vinyl Acetate), (PEVA) | 30 |
| | PGA (micropulv.) | 140 |

The blend was removed from the rubber mill in one uniform sheet. While the blended sheet was still hot from the mill, it was cut into 8 approximately equal parts to be used for pressing into thinner sheets of film.

A disc was compressed from one of the pieces of Blend #2 between stainless steel caul plates and shim stock of 24 mils. thick. A disc was formed which measured approximately 10.5" diameter and 24 mils thick (0.024") and weighed 49 g.

After extracting the PMMA (Acrylite™) polymer phase with solvent, the resultant microporous film structure is a composite of the remaining unextractable polymer components namely, PGA (70 wt. %), PEVA (15 wt. %), and fibrillar PTFE (15%).

Example 10

A micropulverized PGA polymer biocomponent blend, similar to Example 9, is blended on a rubber mill pre-heated to about 160-170° C. The composition of the blend is as follows:

| Blend No. | Components | Wt. g. |
|---|---|---|
| 3 | Acrylite™ H-12 | 200 |
| | PTFE (30B susp.) | 10 (12 ml.) |
| | PGA (micropulv.) | 190 |

The blend was removed from the rubber mill in one uniform sheet. While the blended sheet was still hot from the mill, it was cut into 8 approximately equal parts to be used for pressing into thinner sheets of film.

One plaque from blend #3 above was compressed between stainless steel caul plates and shim stock (44 mil. or 0.044") for about 15 min. at 350-360° F at a pressure of 30 tons on a 6 inch diameter ram. The platens measured 12 inch x 12 inch. They were removed and cooled under pressure for about 5 min. A disc was formed which measured approximately 7.5 inches diameter and approximately 43 mils thick (0.043") weighing 52 g.

After extruding the PMMA (Acrylite™) polymer phase with solvent, the resultant microporous film structure is a composite of the remaining unextractable polymer components, namely, PGA (95 wt. %), and fibrillar PTFE (5 wt %).

Example 11

The density of sheets compressed to a thickness of 24 mils (0.024") from the blends in Examples 8 to 10 are as follows:

| Example 8 | Sheet #1 | 1.28g/cc |
|---|---|---|
| | Sheet #2 | 1.33g/cc |
| Example 9 | Sheet #1 | 1.26g/cc |
| | Sheet #2 | 1.26g/cc |
| Example 10 | Sheet #1 | 1.32g/cc |
| | Sheet #2 | 1.32g/cc |

The above sheets were extracted by immersing them four times in acetone, at 16 hours each, replacing the acetone after each 16 hour immersion with clean fresh acetone. The sheets were then washed in methanol, pressed between blotter paper, and air dried.

Example 12

The following is the PMMA extraction data for the sheets of Examples 8 to 10.

| | | | Weight After Acetone Extraction | Weight Before Acetone Extraction | % PMMA Extracted |
|---|---|---|---|---|---|
| Example | 8: | #1 | 30.34g. | 61.82g. | 49.07 |
| | | #2 | 21.71g. | 43.01g. | 50.47 |
| Example | 9: | #1 | 23.32g. | 47.2g. | 49.4 |
| | | #2 | 23.68g. | 47.9g. | 49.44 |
| Example | 10: | #1 | 25.99g. | 53.2g. | 48.9 |
| | | #2 | 27.67g. | 56.4g. | 49.06 |

Example 13

Sheets #2 from Example 11 above were cut into pieces measuring 1/2 inch X 1 inch and were sterilized with ethylene oxide gas in preparation for animal implant testing.

Samples of these three types of microporous composite membrane structures were evaluated for material integrity, adsorption, and tissue reaction when implanted subcutaneously in rabbits for up to 6 months.

Two samples of each membrane were implanted subcutaneously on each side of the abdominal midline in each of five adult New Zealand albino rabbits. Test intervals were scheduled for 14 days, 1, 2, 3 and 6 months. The rabbit scheduled for six months received only one of the Example 11 samples.

At the end of the test interval one sample from each membrane composition was resected en bloc and prepared for microscopic examination. The other sample was removed and subjected to manual manipulation to determine its consistency.

Results

Morbidity and Mortality:

Four of the animals were helthy at term. One animal developed ulcerated foot and heel pads and was sacrificed at 65 days instead of the scheduled 3 months. This problem was not associated with the implant materials.

Summary and Conclusions

Initially, all three membrane types were pliable and resistant to stretch. By 14 days and thereafter the bicomponent materials were pliable but "taffy" like. the tricomponent membrane maintained its integrity throughout the study. No adverse gross tissue response nor gross evidence of absorption was seen at any interval.

Microscopically the membranes stimulated a slight to moderate phagocytic response and thin fibrous capsule formation. The least response was seen with the tricomponent membrane. Material degradation appears to be due more to phagocytosis than to hydrolysis.

Due to the material integrity and lack of tissue reaction seen with the 15% PTFE/15% PEVA/70% PGA membranes, this material is preferred. Possible uses for the material are as a soft tissue patch and/or a segmental replacement of a hollow organ. This material, either alone or in combination with other absorbable (e.g., GLY/TMC) and/or nonabsorbable (e.g., a polybutester) materials, may also be useful to prevent or reduce tissue adhesions and/or as a pericardial patch.

The gross observations are shown in Table VIII.

## TABLE VIII

### Tissue Reaction

| Sample ID | 14 Days | 30 Days | 59 Days | 65 Days | 184 Days |
|---|---|---|---|---|---|
| Example 9 | none | none | none | none | none |
| Example 10 | none | none | none | none | none |
| Example 11 | none | slight neo-vascularity | none | none | none |

### Consistency

| Sample ID | 14 Days | 30 Days | 59 Days | 65 Days | 184 Days |
|---|---|---|---|---|---|
| Example 9 | stretchy "taffy-like" pliable | same | same | same | same |
| Example 10 | no stretch pliable strong | same | same | same | same |
| Example 11 | no stretch pliable fibrous on breaking | stretchy pliable | stretchy "taffy-like" weak | very "taffy-like" | same |

## Claims

1. A composite material for use with mammalian tissue comprising:
    a) an unsintered, microfibrillar, nonabsorbable bicompatible component prepared from polytetrafluoroethylene, and

b) a bioabsorbable component manufactured from a polymer prepared from one or more monomers selected from the group consisting of lactides, carbonates, oxalates and lactones, and optionally

c) a non-absorbable, bicompatible thermoplastic component manufactured from a polymer which is liquid at a temperature from about 150 to 200°C and solid at ambient temperature, and which provides additional integrity to the unsintered component.

2. The material of claim 1 wherein the bioabsorbable component, and optionally the nonabsorbable, thermoplastic component is enmeshed in the pores of the unsintered component.

3. The material of claim 1 wherein the bioabsorbable component is in particulate form.

4. The material of claim 3 wherein the particulate bioabsorbable component is mocropulverized.

5. A composite material for use with mammalian tissue comprising a first part consisting of the material of claim 1 or 2 or 3 or 4; and a second part affixed to at least one side of the first part, the second part comprising a bioabsorbable textile reinforcement component, the bioabsorbable reinforcement component being woven or knitted, and manufactured from the same or a different polymer than the bioabsorbable component of the first part.

6. The material of claim 5 wherein the textile reinforcement material is affixed to both sides of the first part.

7. The material of claim 6 wherein said reinforcement material is laminated to said first part.

8. The material of claim 1 or 2 or 3 or 4 wherein the bioabsorbable component is manufactured from a homopolymer or copolymer and wherein the lactide is selected from the group consisting of glycolide and 3,6-dimethyl-1,4-dioxane-2,5-dione; the carbonate is 1,3-dioxan-2-one; and the lactone is selected from the group consisting of e-caprolactone and 1,4-dioxan-2-one.

9. The material of claim 1 or 2 or 3 or 4 wherein the composite material is in the form of a sheet or hollow tube.

10. The material of claim 8 wherein the nonabsorbable, thermoplastic component is poly-(ethylenevinylacetate).

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-334046 (AMERICAN CYANAMID COMPANY) <br> * claims 1-10 * <br> --- | 1-10 | A61L27/00 <br> A61L31/00 <br> A61L15/26 |
| X | WO-A-8804557 (ALLIED CORPORATION) <br> * page 3, lines 8 - 16 * <br> * page 4, lines 1 - 10 * <br> --- | 1, 9 | A61L15/24 <br> A61L15/64 |
| A | EP-A-334024 (AMERICAN CYANAMID COMPANY) <br> * page 3, lines 36 - 50; claims 1-10 * <br> --- | 1, 9 | |
| A | EP-A-159502 (AMERICAN CYANAMID COMPANY) <br> * page 1, lines 1 - 12 * <br> * page 5, lines 25 - 28 * <br> --- | 1 | |
| A | EP-A-202444 (AMERICAN CYANAMID COMPANY) <br> --- | | |
| A | EP-A-269449 (BAXTER TRAVENOL LABORATORIES, INC.) <br> ----- | | |
| | | | **TECHNICAL FIELDS SEARCHED (Int. Cl.5)** <br><br> A61L |

The present search report has been drawn up for all claims

| Place of search | Date of completion of the search | Examiner |
|---|---|---|
| THE HAGUE | 30 MAY 1991 | ESPINOSA Y CARR |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................
& : member of the same patent family, corresponding document

EPO FORM 1503 03.82 (P0401)